# EUROPEAN PATENT SPECIFICATION

(11) **EP 2 712 570 B1**
(45) Date of publication and mention of the grant of the patent: **26.10.2016**
(21) Application number: 13184876.4
(22) Date of filing: 18.09.2013
(51) Int. Cl.: A61B 34/10, G06F 19/00

(54) **Operation support system for confirming a surgical site**
Operationsunterstützungssystem zur Bestätigung einer Operationsstelle
Système de support d'opération pour confirmation d'un site chirurgical

(30) Priority: 28.09.2012 JP 2012217140
(43) Date of publication of application: 02.04.2014
(73) Proprietor: Nihon Kohden Corporation, Shinjuku-ku Tokyo (JP)
(72) Inventor: Kasuya, Hiromitsu, Tokyo (JP); Muneshima, Rie, Tokyo (JP)
(74) Representative: Ahner, Philippe

(56) References cited:
- WO-A1-2007/017642
- US-A1- 2006 078 860
- US-A1- 2006 149 296
- US-A1- 2010 082 368

## Description

### BACKGROUND OF THE INVENTION

The present invention relates to an operation support system which prevents a surgical site from being mistaken for another part (wrong-site surgery) at the time of a surgery.

Usually, for a management of the surgery, a method is used in which personal information including, first of all, the name or the sex distinction of the patient is written on a sheet called an order sheet, an operative procedure is written thereon together with a surgical position by characters as surgery information, and the order sheet is referred to apply a mark to the surgical site of the patient in an operation room.

However, even when the order sheet on which the characters are written is visually observed and recognized at the time of the surgery, a situation is concerned about that the surgical site is mistaken for another part. Especially, are reported that the patient himself or herself is mistaken for another patient, a right organ, a right part to be cut out and a right part to be extracted are respectively mistaken for a left organ, a left part to be cut out and a left part to be extracted, and vice-versa, and a misunderstanding based on a surgical position that the patient has to take in the operation room. Thus, an improvement is required.

As compared with the above-described method, a system is known in which surgery schedule information is electronically formalized and managed; however, the above-described system cannot be used as a countermeasure for a mistake surgical site for another part (see patent literature 1).

Further, in order to simulate a medical treatment and medical conduct such as a surgery, a system using a three-dimensional graphics is known (see patent literature 2). However, this system is used for training, learning or an experiment to the end. Thus, in this system, a surgical position or a surgical site cannot be recognized immediately before the surgery.

[Patent Literature 1] JP-A-2007-122174
[Patent Literature 2] JP-A-H11-219100

US 2006/0149296 discloses an apparatus and method for preventing wrong-side surgery.

US 2006/078860 discloses an operation support system including a confirmation board apparatus comprising an image of a human body that is visibly divided into sections; and a mechanism for visibly highlighting individual sections of the image.

### SUMMARY

The present invention is devised by considering such existing circumstances of a medical practice as described above, and it is an object of the present invention to provide an operation support system which can reduce medical errors, for instance, a patient himself or herself, an organ, a part to be cut out or a part to be extracted is mistaken for another patient, another organ, another part to be cut out or another part to be extracted.

### [Means for Solving the Problems]

It is therefore an aspect of the invention to provide an operation support system according to claim 1 for confirming a surgical site and a surgical position. The operation support system includes:
an input unit which inputs surgery information including an operative procedure, a surgery on right or left side and the surgical position,
a model image forming unit which applies a mark to the surgical site of the corresponding surgical position in accordance with the inputted surgery information to form a human body model image;
a display unit which displays the surgery information and the image and
a display control unit which displays the human body model image formed by the model image forming unit on the display unit.

The operation support system may further include:
a result input unit which inputs a compared result of the surgical position and the surgical site of a patient in a surgery and the human body model image displayed on the display unit, wherein the display control unit allows the inputted compared result to be displayed on the display unit.

The input unit may include a unit which changes an orientation of the human body model image displayed on the display unit, and,
when a change of an orientation is inputted, the model image forming unit forms a human body model image the orientation of which is changed so as to meet the inputted change of the orientation.

The operation support system may further include:
an image recording unit which records an image of the patient to obtain a recorded image, wherein the display control unit displays the recorded image on the display unit together with the human body model image.

### BRIEF DESCRIPTION OF THE DRAWINGS

Fig. 1 is a block diagram showing one exemplary embodiment of a medical operation support system according to the present invention.
Fig. 2 is a flowchart showing a behavior of the exemplary embodiment of the medical operation support system according to the present invention.
Fig. 3 is a diagram showing one example of a display appearing in the behavior of the exemplary embodiment of the medical operation support system according to the present invention.
Fig. 4 is a diagram showing one example of a display appearing in the behavior of the exemplary embodiment of the medical operation support system according to the present invention.
Fig. 5 is a diagram showing one example of a display appearing in the behavior of the exemplary embodiment of the medical operation support system according to the present invention.
Fig. 6 is a diagram showing one example of a human body model image displayed in the behavior of the exemplary embodiment of the medical operation support system according to the present invention.
Fig. 7 is a diagram showing one example of human body model images provided in the behavior of the exemplary embodiment of the medical operation support system according to the present invention.
Fig. 8 is a diagram showing another example of human body model images provided in the behavior of the exemplary embodiment of the medical operation support system according to the present invention.
Fig. 9 is a diagram showing a human body model image the orientation of which is changed in the behavior of the exemplary embodiment of the medical operation support system according to the present invention and an orientation of an actual patient.
Fig. 10 is a diagram showing a display example in which a recorded image of the patient in a surgery and the human body model image are displayed on one screen.

### DETAILED DESCRIPTION OF EMBODIMENTS

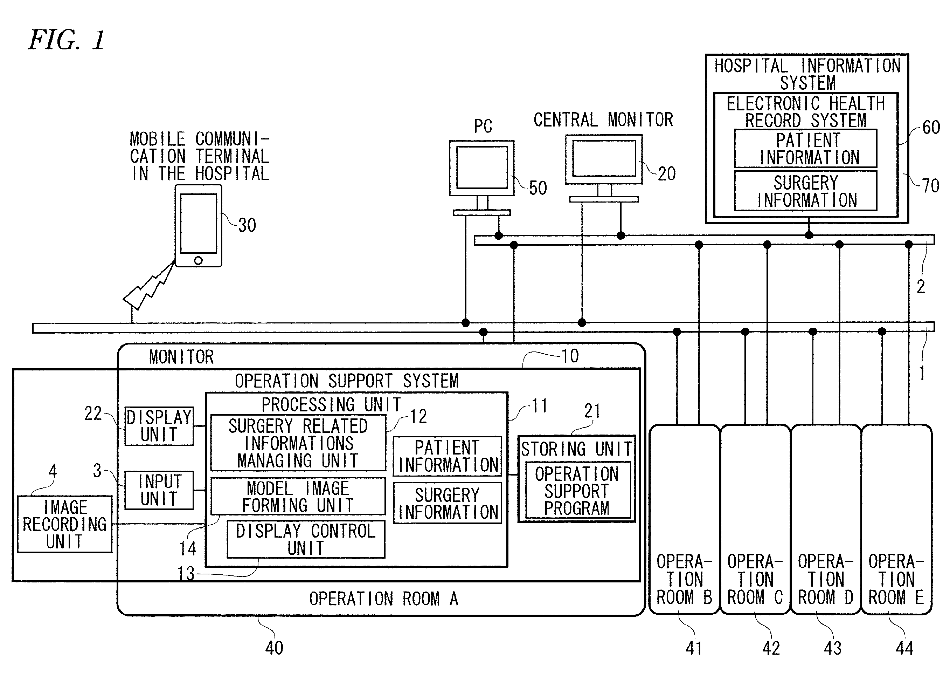

Now, an exemplary embodiment of an operation support system of the present invention will be described below by referring to the attached drawings. In the drawings respectively, the same component elements are designated by the same reference numerals and a duplicated explanation is omitted. Fig. 1 is a block diagram showing the exemplary embodiment of the operation support system. The same systems as the operation support system 10 are respectively provided in monitors 40 to 44 which are respectively provided in operation rooms A to E, a central monitor 20, a mobile communication terminal 30 in a hospital and a personal computer 50 (the monitors 40 to 44, the central monitor 20, the mobile communication terminal 30 and the personal computer 50 are generally referred to as devices, hereinafter). Reference numeral 60 designates a hospital information system which includes various systems relating to a hospital as well as an electronic health record system 70 in which patient information, surgery information or the like is stored. The hospital information system 60 and the devices send and receive information through networks 1 and 2 in the hospital. The devices respectively send and receive information through the network 1. The kinds and the number of the above-described devices in this embodiment are merely one example.

Now, the operation support system 10 will be described in detail. The operation support system 10 provided in each of the monitors 40 to 44 of the operation rooms is mainly used in a surgery performed in the operation room. The operation support systems 10 provided in the central monitor 20, the mobile communication terminal 30 and the personal computer 50 can be respectively used in any of the operations performed in the operation rooms A to E. Further, the central monitor 20, the mobile communication terminal 30 and the personal computer 50 can mutually monitor a usage conditions of the operation support systems 10 respectively used by them. In the present exemplary embodiment, will be described the operation support system 10 of the monitor 40 installed in the operation room A where the surgery is actually performed. In the block diagram of the monitor 40 shown in Fig. 1, a block diagram of a physiological information monitoring function of a patient is omitted.

The operation support system 10 includes a processing unit 11, a storing unit 21, an input unit 3, a display unit 22 and an image recording unit 4. These units are not provided as new hardware except the image recording unit 4 and are added as new functions by using hardware provided in the monitor 40. The above-described matter is likewise applied to the monitors 41 to 44. The operation support systems 10 of the central monitor 20, the mobile communication terminal 30 and the personal computer 50 are the same as that of the monitor 40 except that the image recording unit 4 is not provided.

The processing unit 11 is composed of a CPU. To the processing unit 11, are connected the storing unit 21 which stores programs or data, the display unit 22 composed of an LCD, the input unit 3 composed of a keyboard or a touch panel and the image recording unit 4.

The storing unit 21 stores an operation support program which is read and executed by the processing unit 11. The processing unit 11 includes a surgery related informations managing unit 12, a model image forming unit 14 and a display control unit 13 as functions for carrying out processes when the operation support program is executed.

The surgery related informations managing unit 12 collects and holds various information concerning the surgery of the patient from the electronic health record system 70, and collects and holds the patient information and the surgery information such as an operative procedure, an surgery side (a right or a left side), a surgical position or the like. Necessary information may be not only inputted from the electronic health record system 70, but also manually inputted from the input unit 3.

The model image forming unit 14 applies a mark to a surgical site of the corresponding surgical position in accordance with the stored information such as the operative procedure, the surgery side (the right or the left side), the surgical position or the like to form a human body model image. The display control unit 13 displays the human body model image formed by the model image forming unit 14 on the display unit 22. The human body model image can be also inspected on the central monitor 20, the mobile communication terminal 30 and the personal computer 50.

The image recording unit 4 records an image of the patient in the surgical position to obtain the image of the patient. The recorded image of the patient is converted into image information which can be processed by a computer and fetched to the processing unit 11. The display control unit 13 displays the recorded image of the patient together with the human body model image on the display unit 22 and display units (describe them as a "display unit 22 or the like", hereinafter) of the central monitor 20, the mobile communication terminal 30 and the personal computer 50. Accordingly, on the display unit 22 or the like, the recorded image of the patient in a surgical position and the human body model image are displayed.

As described above, on the display unit 22 or the like, the image of a human body model is displayed, and, for the actual patient in the surgery, the recorded image of the patient can be recognized on the display unit 22 or the like. Thus, each healthcare personnel compares a surgical site and the surgical position of the actual patient in the surgery (the actual patient in the operation room A) with those of the human body model image displayed on the display unit 22 or the like, so that he or she can input a compared result from the input unit 3 and input units of the central monitor 20, the mobile communication terminal 30 and the personal computer 50. The display control unit 13 and display control units of the central monitor 20, the mobile communication terminal 30 and the personal computer 50 display the compared result on the display units. Further, the devices may be respectively provided with generating units of an alarm such as an alarm sound or a flicker of light as well as the display units. When the compared result shows a non-coincidence, the alarm generating units may generate an alarm such as the alarm sound.

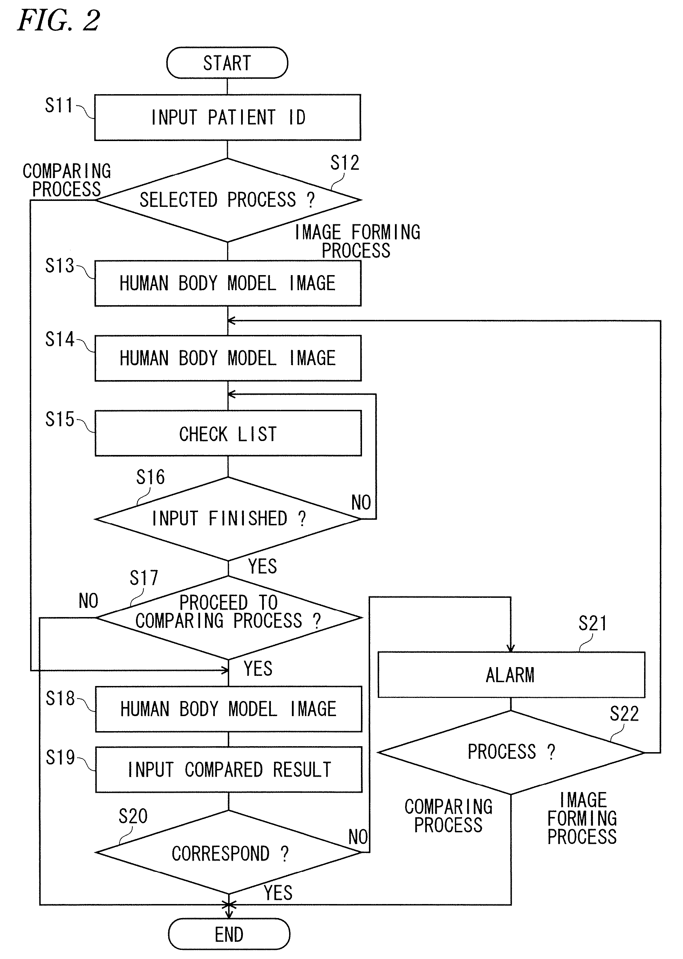

A behavior and an operation of the operation support system constructed as described above will be described below in accordance with a flowchart shown in Fig. 2. In an explanation, initially, a basic behavior and operation will be described that the healthcare personnel forms the human body model image. Then, a behavior and operation will be described that each healthcare personnel relating to the surgery recognizes the surgical site and the surgical position by using the formed human body model image. Finally, a behavior and operation will be described that the healthcare personnel corrects the formed human body model image.

When a program of the operation support system is started, initially, a patient ID input is guided. Thus, the patient ID is inputted by the input unit 3 (step S11). Then, in step S12, is selected either a human body model image forming and correcting process (an image forming process) or a comparing process that the formed human body model image is compared with the recorded image of the patient in the surgical position that is recorded by the image recording unit 4 or the actual surgical position of the patient.

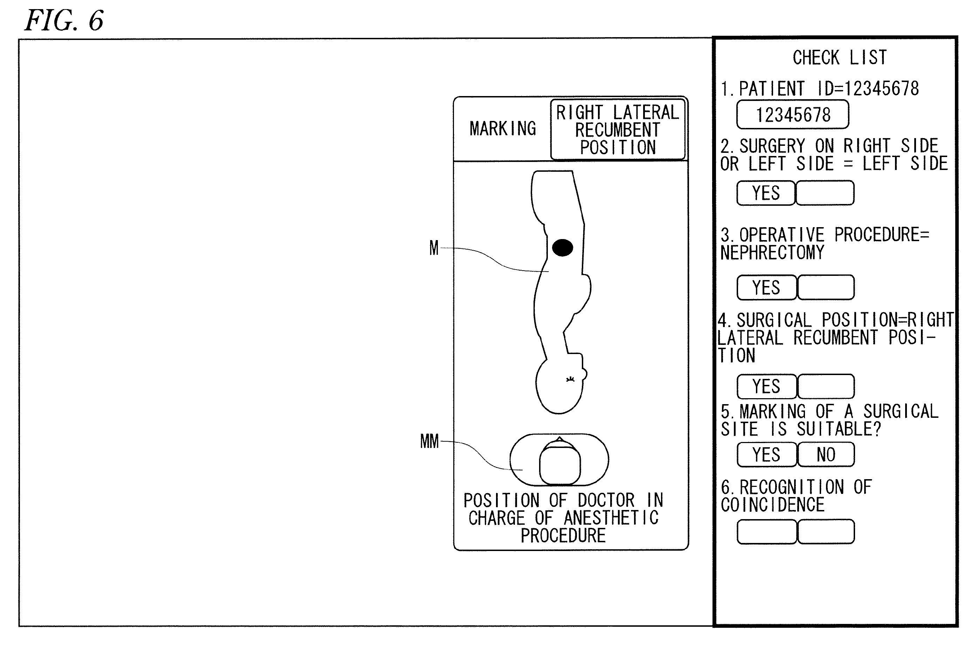

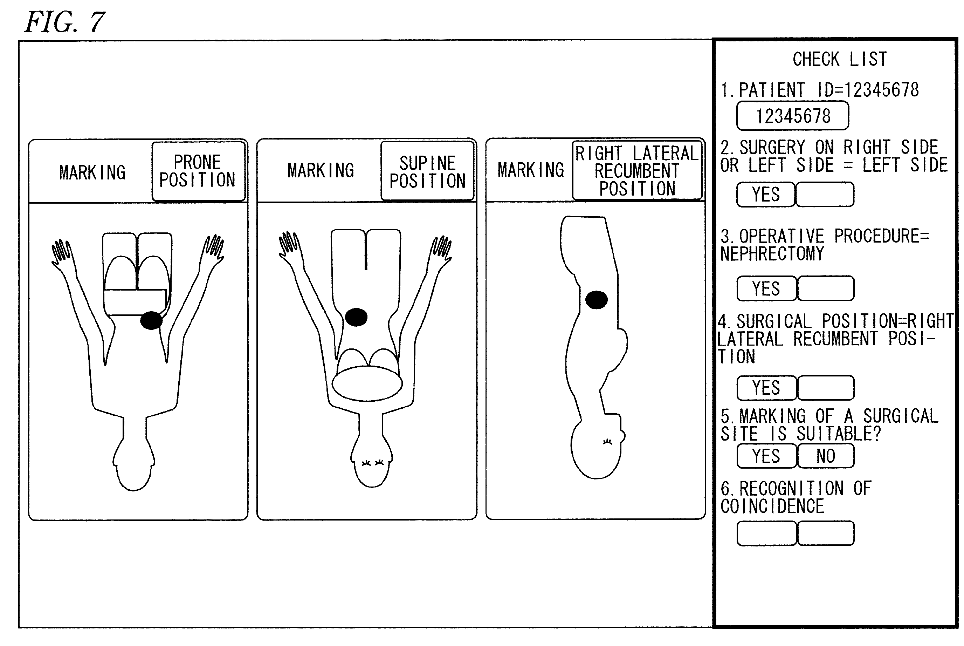

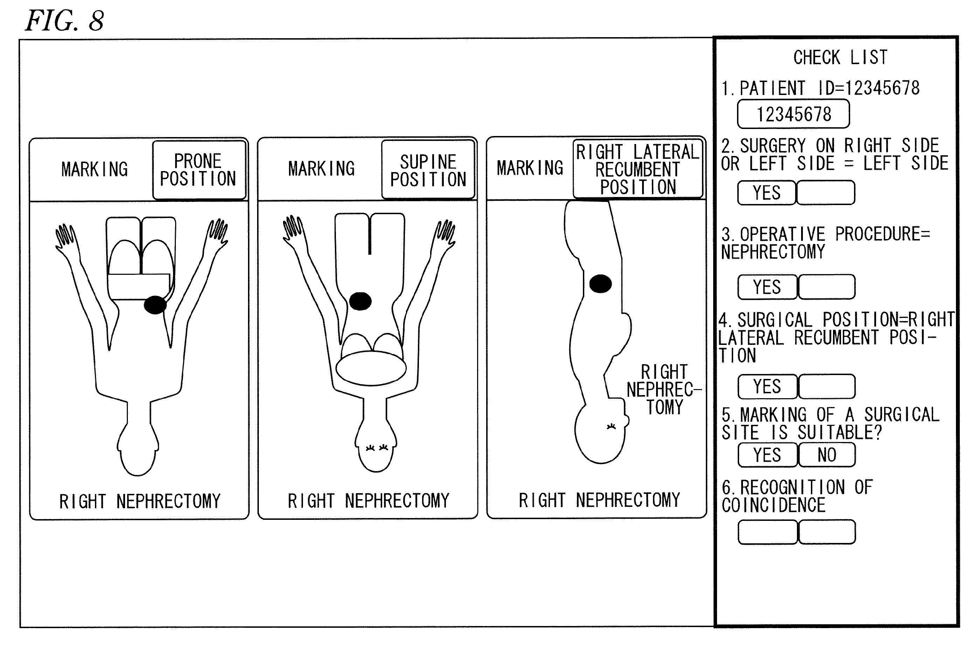

In order to form the human body model image, initially, when the image forming and correcting process (the image forming process in the step S12) is selected, in step S13, the forming process of the human body model image is carried out. The human body model image is formed in such a way that the model image forming unit 14 applies the mark to the surgical site of the corresponding surgical position in accordance with the stored information such as the operative procedure, the surgery side (the right or the left side), the surgical position or the like. As for the formed human body model image, as shown in Fig. 6, the human body model image M is displayed on the display unit 22 together with a check list by the display control unit 13 (step S14). Sign MM designates a doctor in charge of anesthetic procedure. As for the human body model image, different human body model images are respectively prepared for a man and a woman and selected in accordance with gender-based information held by the surgery related informations managing unit 12. Fig. 6 shows the human body model image M of a woman. Further, the human body model image includes a three-dimensionally displayed model shown in Fig. 7 and a two-dimensionally displayed model shown in Fig. 8 and they can be changed and used as required.

Then, in step S15, inputs to items of the check list are respectively carried out. In the check list, subsequent to a display of the patient ID inputted in the step S11, are displayed check items, for instance, the "surgery on right side or left side", the "operative procedure", the "surgical position" and "a marking of a surgical site is suitable?". As for the check items of the "surgery on right side or left side", the "operative procedure", the "surgical position", are presented, in the right sides of the check items, displays based on the operative procedure, the surgery side (the right or the left side) and the surgical position which are stored as the information of the patient. The check items have respectively check boxes of "Yes" and "No". For the items of the check list respectively, when the displayed check item is right, "Yes" is selected, and when the displayed check item is wrong, "No" is selected. When "Yes" is selected, the check item is fixed.

Then, in step S16, it is decided whether or not the input of the check list is finished. The decision is made depending on whether or not, for instance, an "input completion" key which is not shown in the drawing is clicked. As long as the input completion key is not clicked, the check list of the step S15 may be continuously in input enabling state.

In the input of the check list in the step S15, when there is an item among the items which is not displayed or is incorrectly displayed, the human body model image is incomplete or incorrect. In this case, in the item in question, "No" is selected. Here, when the item is corrected, if the item in question of the check list is clicked, a correction screen is displayed, which will be described below.

When the "input completion" key which is not shown in the drawing is clicked (step S16 (YES)), in step S17, it is determined whether or not the procedure proceeds to the comparing process where the human body model image is compared with the recorded image of the patient or the actual patient. When the procedure does not proceed to the comparing process (NO), the operation support system is finished. Whether the procedure "proceeds" or "does not proceed" to the comparing process can be selected by clicking a "comparing process" key or a "completion" key which is not shown in the drawing. Even when the input of the check list is not completed, the operation support system can be finished by the steps S16 and S17.

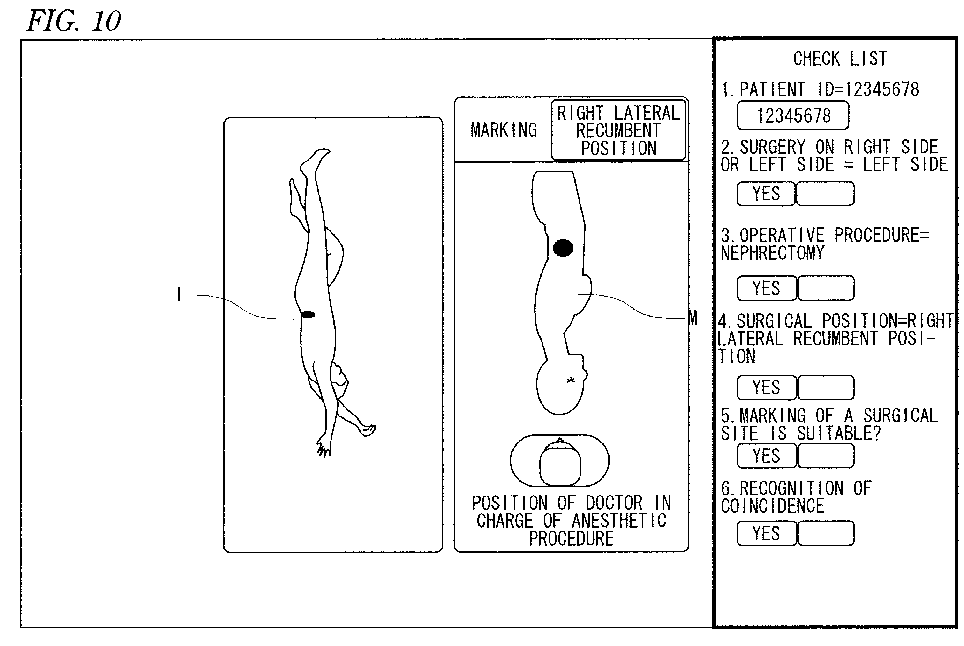

When the procedure proceeds to the comparing process (the step S17 (YES)), in step S18, the human body model image M and the recorded image I of the patient in the surgical position are arranged side by side and displayed together with the check list in the display unit 22 as shown in Fig. 10.

Then, the human body model image M is compared with the recorded image I of the patient or the actual patient to decide whether or not they correspond to each other and input a result of decision from the input unit 3 (step 19). As for the input of the result, in a section of "recognition of coincidence" of the check list shown in Fig. 10, when the compared result shows a coincidence, "Yes" is clicked, and when the compared result shows a non-coincidence, "No" is clicked (a result input unit). The healthcare personnel compares the human body model image with the recorded image of the patient or the actual patient in the steps S18 and S19, so that he or she can sufficiently recognize the surgical site and the surgical position.

In step S20, when the inputted compared result is determined and the compared result shows the coincidence (YES), the display unit 22 displays the coincidence to finish the operation support system. When the inputted compared result shows the non-coincidence (NO), the display unit 22 displays that the compared result does not show the coincidence in step S21 to generate the alarm such as the alarm sound and guide the healthcare personnel to make a correction. In step S22, it is determined whether the process selected in the step S12 is the image forming process or the comparing process. In the above-described explanation, since the selected process is the image forming process, the procedure returns to the step S14 to display the human body model image so that the human body model image may be corrected. The correcting process will be described below.

Now, a flow of processes will be described that the healthcare personnel relating to the surgery recognizes the surgical site and the surgical position by using the human body model image formed as described above. The recognition can be carried out by the monitor 40, the central monitor 20, the mobile communication terminal 30 in the hospital and the personal computer 50. Units of the central monitor 20, the mobile communication terminal 30 and the personal computer 50 which respectively correspond to the units of the monitor 40 are designated as the units of the monitor 40 "or the like".

The program of the operation support system is started to input the patient ID in the step S11 shown in Fig. 2. Then, in the step S12, when the comparing process is selected that the formed human body model image is compared with the recorded image of the patient in the surgical position that is recorded by the image recording unit 4 or the actual patient (the step S12 (the comparing process)), the procedure proceeds to the step S18. In the step S18, the human body model image M and the recorded image I of the patient in the surgical position are arranged side by side and displayed together with the check list in the display unit 22 as shown in Fig. 10.

Then, the human body model image M is compared with the recorded image I of the patient or the actual patient to decide whether or not they correspond to each other and input a result from the input unit 3 or the like(the step 19). As for the input of the result, in the section of "recognition of coincidence" of the check list shown in Fig. 10, when the compared result shows a coincidence, "Yes" is clicked, and when the compared result shows a non-coincidence, "No" is clicked (the result input unit). The healthcare personnel compares the human body model image with the recorded image of the patient or the actual patient in the steps S18 and S19, so that he or she can completely recognize the surgical site and the surgical position.

In the step S20, when the inputted compared result is determined and the compared result shows the coincidence (YES), the coincidence is displayed on the display unit 22 to finish the operation support system. When the inputted compared result does not show the coincidence (NO), the compared result showing the non-coincidence is displayed on the display unit 22 in the step S21 to generate the alarm such as the alarm sound. In the step S22, it is determined whether the process selected in the step S12 is the image forming process or the comparing process. In the above-described explanation, since the selected process is the comparing process, the operation support system is finished. According to the above-described processes, even when one of healthcare personnel using the operation support system perceives a difference between the human body model image and the recorded image of the patient or the actual patient, the difference is informed of by the alarm. Thus, the healthcare personnel can recognize the compared result again, so that a medical error can be prevented.

Now, the behavior and operation will be described that the healthcare personnel corrects the formed human body model image. As described above, in the input of the check list in the step S15, when there is an item among the items which is not displayed or is incorrectly displayed, the human body model image is incomplete or incorrect. In a below-described explanation, a situation will be described that all other items than the patient ID are corrected. In the check list shown in Fig. 6, when the item of the check list is clicked to correct the item in question, the correction screen is displayed. When the operation support program is started again after the operation support system is finished once, the items can be corrected in the step S15.

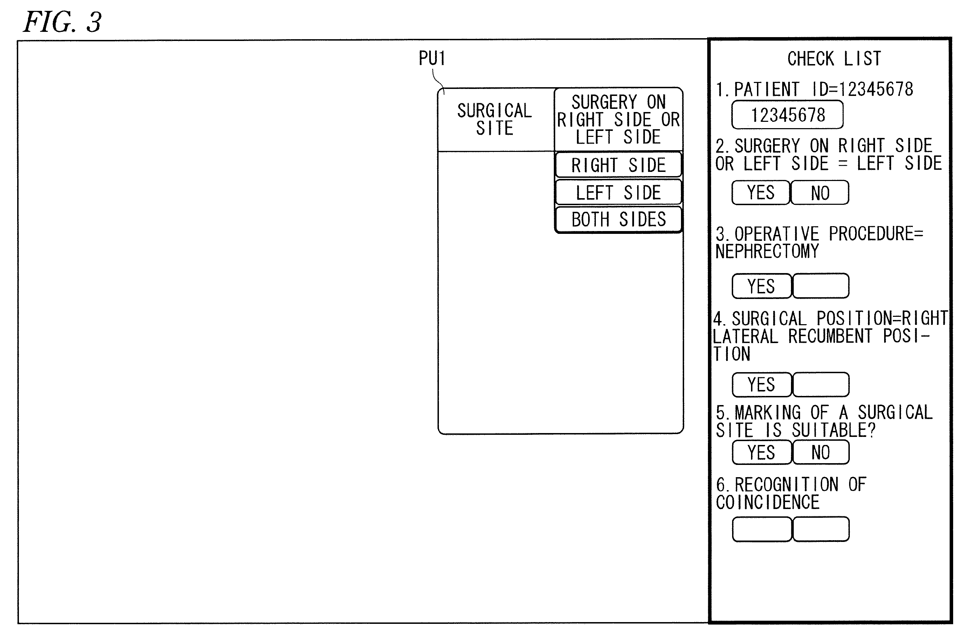

In Fig. 6, when the "surgery on right side or left side" of the check list is corrected, for instance, a point of the "surgery on right side or left side" is clicked. Then, a pop up screen PU1 is displayed in the left of a check list section as shown in Fig. 3 to display selection buttons of a "right side", a "left side" and "both sides" so that the surgery on which side may be selected as the surgical site. When the selection button of an intended surgical site is clicked and a selection button of "Yes" is selected, the intended surgical site is determined. When the surgery on right or left side is necessary to be changed, a selection button of "No" is clicked, the surgical site can be changed.

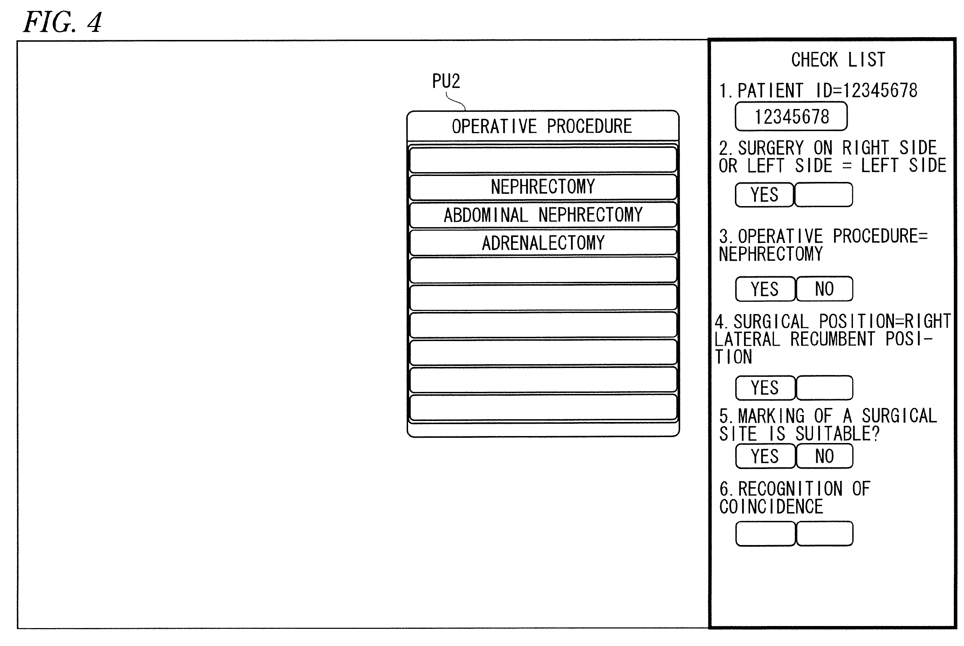

When the correcting process is continuously carried out, for instance, if a point of the "operative procedure" of the check list shown in Fig. 3 is clicked, a pop up screen PU2 is displayed in the left of the check list section as shown in Fig. 4 to display selection buttons of exemplifications of an "nephrectomy", an "abdominal nephrectomy", an "adrenalectomy" or the like as the operative procedure. It is to be understood that the exemplifications displayed herein merely show examples. When the selection button of a intended operative procedure is clicked and a selection button of "Yes" is clicked, the intended operative procedure is determined. When the operative procedure is necessary to be changed, a selection button of "No" is clicked so that the operative procedure may be changed.

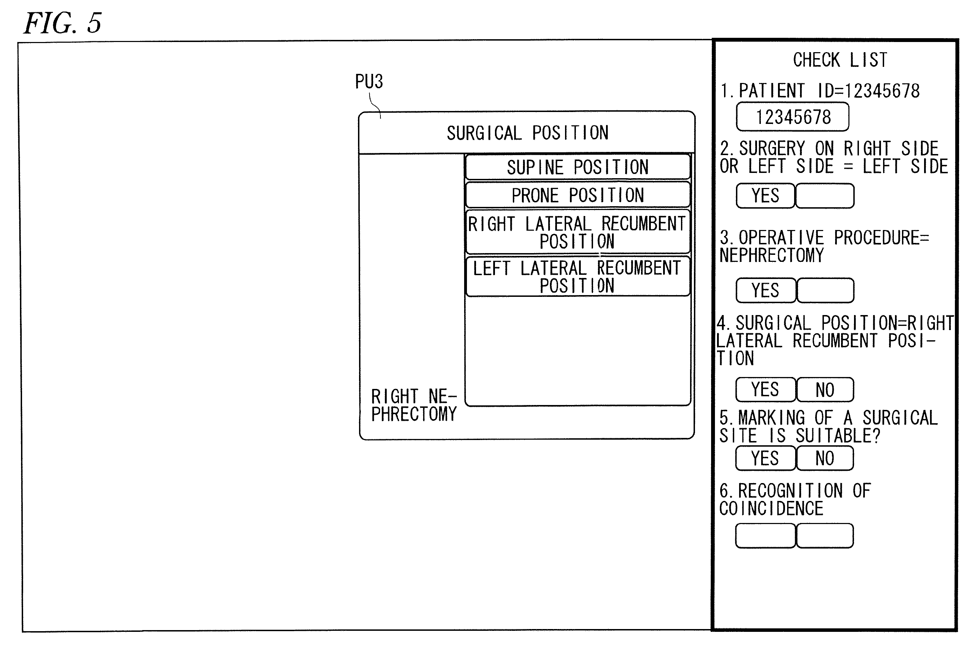

When the correcting process is continuously carried out, for instance, if a point of the "surgical position" of the check list shown in Fig. 4 is clicked, a pop up screen PU3 is displayed in the left of the check list section as shown in Fig. 5 to display selection buttons of "supine position", "prone position", "right lateral recumbent position", "left lateral recumbent position" or the like as the surgical position. When the selection button of an intended surgical position is clicked and a selection button of "Yes" is clicked, the intended surgical position is determined.

When the correcting process is completed as described above and the "input completion" key not shown in the drawing is clicked, the procedure proceeds to the step S17 shown in Fig. 2.

In the operation support system, an orientation of the human body model image can be changed. The input unit 3 can input an orientation changing instruction for changing the orientation of the human body model image displayed on the display unit 22. When the orientation changing instruction is inputted, the model image forming unit 14 forms the human body model image the orientation of which is changed so as to meet the input.

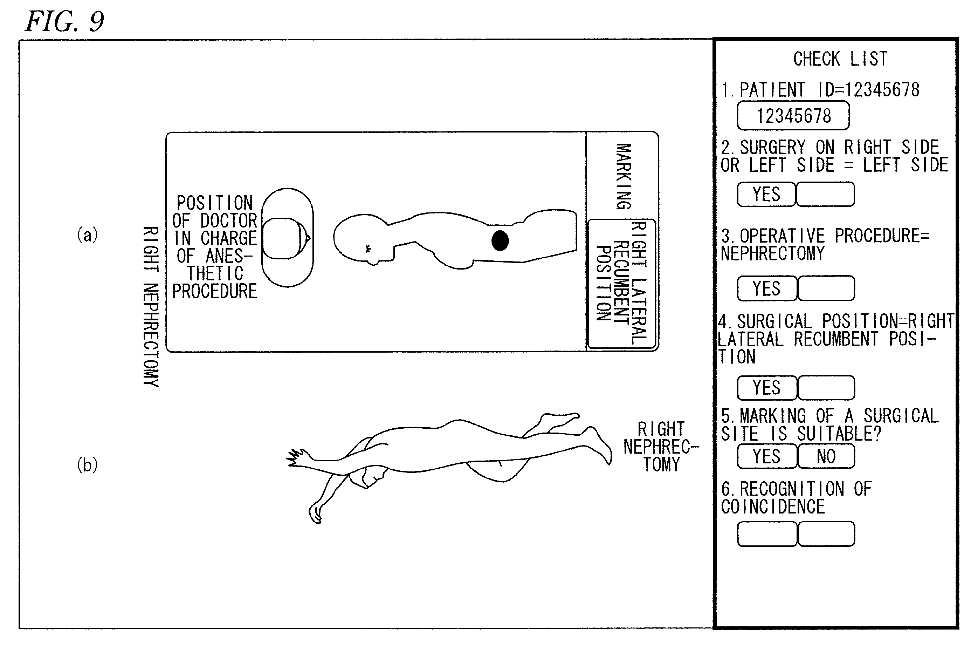

The human body model image the orientation of which is changed as described above is displayed on the display unit 22 by the display control unit 13. Fig. 9(a) shows an example that the human body model image on the right lateral recumbent position changes its orientation so that the head is located in the left side. This display is useful when a person such as an operating surgeon who uses the operation support system of the present exemplary embodiment compares and recognizes the patient (Fig. 9(b)) visually observed from his or her position in surgery with the surgical site or the marking position of the human body model image formed by the operation support system. In the display in such a way, a probability of an erroneous recognition can be lowered.

In the display unit, physiological information can be displayed together with the human body model image. Namely, to the patient whom the surgery is performed, measuring units not shown in the drawing are attached which measure physiological information such as SpO2, blood pressure, an electrocardiogram, body temperature or the like. Wave forms or numerical information thereof are displayed on the necessary monitors (the central monitor 20, the mobile communication terminal 30 in the hospital, the monitors 40 to 44 of sections or the like). Thus, the display control unit can display the human body model image or the recorded image of the patient together with the physiological information on the display unit. As for a form of the display, the human body model image or the recorded image of the patient may be overlapped on each other or display areas may be divided and displayed.

In the above-described exemplary embodiment, the operation support system 10 is provided with the image recording unit 4. However, the image recording unit does not need to be provided. In this case, since the recorded image of the patient is not present, each healthcare personnel recognizes the human body model image on the display unit and visually observes the actual patient to compare them with each other and decide and inputs the result from each of the input units.

Further, in above-described exemplary embodiment, the operation support system 10 is provided respectively in the central monitor 20, the mobile communication terminal 30 and the monitors 40 to 44 of the sections; however, the operation support system may be provided merely in devices relating to the surgery. Devices not providing operation support system may be configured so as to use it by downloading the operation support system from the devices providing the operation support system.

Further, a dedicated server for the operation support system may be provided. In this case, the operation support system may be downloaded and used to a device using the operation support system. Further, a device providing the operation support system may be used as it stands alone.

According to the operation support system of the present invention, since the mark is applied to the surgical site of the corresponding surgical position in accordance with the inputted surgery information to form the human body model image and the formed human body model image is displayed, the surgical position and the surgical site can be recognized by the image before the start of surgery, the medical mistakes, for instance, the surgical site are mistaken for another part, can be reduced.

In the operation support system according to the present invention, the compared result of the surgical position and the surgical site of the patient in the surgery with those of the human body model image displayed on the display unit is inputted to display the inputted compared result. Accordingly, when a few persons input the compared results, if the compared result inputted by even only one person is different, an error is immediately understood. Thus, the medical mistakes, for instance, the surgical site is mistaken for another part, can be more assuredly reduced.

In the operation support system according to the present invention, when the change of the orientation is inputted, since the orientation of the human body model image is formed so as to meet the change of the orientation, the human body model image can be formed and displayed so as to meet an orientation of an actual patient. Accordingly, the medical mistakes, for instance, the surgical site is mistaken for another part, can be reduced.

In the operation support system according to the present invention, since the image of the patient is recorded to obtain the recorded image and the recorded image of the patient is displayed on the display unit together with the human body model image, the actual patient is compared with the human body model image on a screen, so that the surgical site or the surgical position can be inspected and recognized. Thus, the medical mistake, for instance, the surgical site is mistaken for another part, can be reduced.

## Claims

1. An operation support system (10) for confirming a surgical site and a surgical position, wherein the operation support system includes:
an input unit (3) configured to input surgery information including an operative procedure, a surgery on right side or left side and the surgical position,
a model image forming unit (14) configured to apply a mark to the surgical site of the corresponding surgical position in accordance with the operative procedure, the surgery on right side or left side and the surgical position included in the inputted surgery information to form a human body model image (M) comprising the mark;
a display unit (22) configured to display the inputted surgery information and the human body model image; and
a display control unit (13) configured to display the human body model image (M) formed by the model image forming unit (14) on the display unit (22).

2. The operation support system according to claim 1, further including:
a result input unit configured to input a compared result indicating whether or not the surgical position and the surgical site in a surgery correspond to the human body model image displayed on the display unit (22), wherein the display control unit (13) allows the inputted compared result to be displayed on the display unit (22).

3. The operation support system according to claim 1 or 2, wherein the input unit (3) includes a unit configured to change an orientation of the human body model image displayed on the display unit (22), and,
when a change of an orientation is inputted, the model image forming unit (14) forms a human body model image (M) the orientation of which is changed so as to meet the inputted change of the orientation.

4. The operation support system according to any one of claims 1 to 3, further including:
an image recording unit (4) configured to record an image of the patient to obtain a recorded image, wherein the display control unit (13) displays the recorded image on the display unit (22) together with the human body model image (M).

## Patentansprüche

1. Betriebsunterstützungssystem (10) zur Begründung eines Chirurgieplatzes und einer Chirurgieposition, wobei das Betriebsunterstützungssystem folgendes aufweist: eine Eingabeeinheit (3), die so gestaltet ist, daß chirurgische Informationen einschließlich einer operativen Vorgehensweise eingegeben werden können sowie eine chirurgische Behandlung auf der rechten Seite oder auf der linken Seite und die chirurgische Position; des weiteren umfassend eine Modellbild-Bildungseinheit (14) zur Anbringung einer Markierung an dem Chirurgieort der entsprechenden chirurgischen Position gemäß der operativen Vorgehensweise, der chirurgischen Behandlung auf der rechten Seite oder linken Seite und der chirurgischen Position als Bestandteil der eingegebenen chirurgischen Information, um ein Modellbild für den menschlichen Körper zu schaffen, das die Markierung aufweist, des weiteren umfassend eine Anzeigeeinheit (22), die dazu dient, die eingegebene chirurgische Information und das Modellbild des menschlichen Körpers darzustellen, und schließlich umfassend eine Anzeige-Steuereinheit (13), die das Modellbild (M) des menschlichen Körpers darstellt, erzeugt von der Modellbild-Formungseinheit (14) auf der Anzeigeeinheit (22).

2. Betriebsunterstützungssystem nach Anspruch 1, ferner **gekennzeichnet durch** eine Ergebniseingabeeinheit, die dazu dient, ein Vergleichsergebnis einzugeben, das anzeigt, ob oder ob nicht die chirurgische Position und der Chirurgieort bei einer chirurgischen Behandlung dem Modellbild des menschlichen Körpers entspricht, das auf der Anzeigeeinheit (22) dargestellt wird, wobei die Anzeige-Steuereinheit (13) ermöglicht, das eingegebene Vergleichsergebnis auf der Anzeigeeinheit (22) darzustellen.

3. Betriebsunterstützungssystem nach Anspruch 1 oder 2, **dadurch gekennzeichnet, daß** die Eingabeeinheit (3) eine Einheit aufweist, die so gebaut ist, daß eine Ausrichtung des Modellbildes des menschlichen Körpers, der auf der Anzeigeeinheit (22) dargestellt wird, geändert werden kann, und dann, wenn eine Änderung einer Ausrichtung eingegeben wird, die Modellbild-Formungseinheit (14) ein Modellbild (M) des menschlichen Körpers herstellt, dessen Ausrichtung so geändert ist, daß sie zu der eingegebenen Änderung der Ausrichtung paßt.

4. Betriebsunterstützungssystem nach einem der Ansprüche 1 bis 3, ferner **gekennzeichnet durch** eine Bildaufzeichnungseinheit (4), die so gebaut ist, daß ein Bild des Patienten aufgezeichnet werden kann, um ein aufgezeichnetes Bild zu erhalten, wobei die Anzeige-Steuereinheit (13) das aufgenommene Bild auf der Anzeigeeinheit (22) zusammen mit dem Modellbild (M) des menschlichen Körpers zeigt.

## Revendications

1. Système d'aide à une opération (10) pour confirmer un site chirurgical et une position chirurgicale, dans lequel
le système d'aide à une opération comprend :
une unité d'entrée (3) configurée pour entrer des informations de chirurgie comprenant une procédure opératoire, une chirurgie de côté droit ou de côté gauche et la position chirurgicale,
une unité de formation d'image de modèle (14) configurée pour appliquer une marque au site chirurgical de la position chirurgicale correspondante conformément à la procédure opératoire, la chirurgie de côté droit ou de côté gauche et la position chirurgicale incluses dans les informations de chirurgie entrées pour former une image de modèle de corps humain (M) comprenant la marque ;
une unité d'affichage (22) configurée pour afficher les informations de chirurgie entrées et l'image de modèle de corps humain ; et
une unité de commande d'affichage (13) configurée pour afficher l'image de modèle de corps humain (M) formée par l'unité de formation d'image de modèle (14) sur l'unité d'affichage (22).

2. Système d'aide à une opération selon la revendication 1, comprenant en outre :
une unité d'entrée de résultat configurée pour entrer un résultat de comparaison indiquant si, oui ou non, la position chirurgicale et le site chirurgical dans une chirurgie correspondent à l'image de modèle de corps humain affichée sur l'unité d'affichage (22), dans lequel l'unité de commande d'affichage (13) permet l'affichage du résultat de comparaison entré sur l'unité d'affichage (22).

3. Système d'aide à une opération selon la revendication 1 ou 2, dans lequel l'unité d'entrée (3) comprend une unité configurée pour changer une orientation de l'image de modèle de corps humain affichée sur l'unité d'affichage (22), et
lorsqu'un changement d'orientation est entré, l'unité de formation d'image de modèle (14) forme une image de modèle de corps humain (M) dont l'orientation est changée de manière à satisfaire au changement d'orientation entré.

4. Système d'aide à une opération selon l'une quelconque des revendications 1 à 3, comprenant en outre :
une unité d'enregistrement d'image (4) configurée pour enregistrer une image du patient pour obtenir une image enregistrée, dans lequel l'unité de commande d'affichage (13) affiche l'image enregistrée sur l'unité d'affichage (22) avec l'image de modèle de corps humain (M).
